# EUROPEAN PATENT SPECIFICATION

(11) **EP 3 584 305 B1**
(45) Date of publication and mention of the grant of the patent: **28.07.2021**
(21) Application number: 18178642.7
(22) Date of filing: 19.06.2018
(51) Int. Cl.: C12M 1/00, C12M 1/02

(54) **MOVABLE CELL INCUBATOR**
BEWEGLICHER ZELLINKUBATOR
INCUBATEUR DE CELLULES MOBILES

(43) Date of publication of application: 25.12.2019
(73) Proprietor: Liu, Lun-Kuang, Hsinchu City (TW)
(72) Inventor: Liu, Lun-Kuang, Hsinchu City (TW); Chen, Yung-Chin, Hsinchu City (TW)
(74) Representative: Lang, Christian

(56) References cited:
- EP-A1- 2 468 844
- EP-A1- 2 698 427
- CN-A- 106 566 772
- TW-A- 201 713 765
- US-A1- 2016 362 650

## Description

### FIELD OF THE INVENTION

The present invention relates to a cell incubator which is compact and is movable easily.

### BACKGROUND OF THE INVENTION

With development of biological technology, cultivating autologous cells is a highlight issue in recently years. To cultivate cells in a sterile environment, the cells are cultivated in an aseptic culture room. However, maintaining culturing condition of the aseptic culture room is difficult.

It is expensive to build a large culture room, for example, the large culture room is established in large hospitals, biochemistry institutes, or organized inspection institutes. However, it will take times for patients to go to these hospitals, biochemistry institutes, and organized inspection institutes.

The present invention has arisen to mitigate and/or obviate the afore-described disadvantages.

### SUMMARY OF THE INVENTION

The primary objective of the present invention is to provide a movable cell incubator which is compact and is movable easily.

Another objective of the present invention is to provide a movable cell incubator which automatically cultivates cells at designated time and quantity

To obtain the above objectives, a movable cell incubator provided by the present invention contains: a body, a first lid, a second lid, and an electric control unit.

The body includes a first internal space, a refrigeration room and an airtight culture room which are located above the first internal space, the refrigeration room is configured to accommodate a cell culture media, and the culture room accommodates at least one cell culture bag.

The first lid airtightly covers the culture room.

The second lid airtightly covers the refrigeration room.

The electric control unit includes a microprocessor, a power module, a digital/analog conversion module defined between the microprocessor and the power module, a heating module for controlling a temperature of the culture room, a cooling module configured to supply cold source to the refrigeration room, a peristaltic pump module, a flow sensing module, a CO₂ detective supply module configured to supply CO₂ to the culture room, and a setting display module exposing and fixed on the first lid.

The peristaltic pump module is aseptically connected between the cell culture media and the at least one cell culture bag by way of multiple conveying tubes, the flow sensing module is serially connected on the multiple conveying tubes, the power module supplies power, and the setting display module is configured to set cell cultivating conditions, the microprocessor operates and controls the cell cultivating conditions, the flow sensing module detects fluid flow, and the peristaltic pump module pumps the cell culture media into the at least one cell culture bag.

### BRIEF DESCRIPTION OF THE DRAWINGS

FIG. 1 is a perspective view showing the assembly of a movable cell incubator according to a preferred embodiment of the present invention.
FIG. 2 is a perspective view showing the assembly of a part of the movable cell incubator according to the preferred embodiment of the present invention.
FIG. 3 is a top plan view showing the assembly of the movable cell incubator according to the preferred embodiment of the present invention.
FIG. 4 is a cross sectional view showing the assembly of the movable cell incubator according to the preferred embodiment of the present invention.
FIG. 5 is another cross sectional view showing the assembly of the movable cell incubator according to the preferred embodiment of the present invention.
FIG. 6 is another perspective view showing the assembly of a part of the movable cell incubator according to the preferred embodiment of the present invention.
FIG. 7 is a bottom plan view showing the assembly of a part of the movable cell incubator according to the preferred embodiment of the present invention.
FIG. 8 is a block diagram view showing a circuit of an electric control unit of the movable cell incubator according to the preferred embodiment of the present invention.
FIG. 9 is also another perspective view showing a part of the assembly of the movable cell incubator according to the preferred embodiment of the present invention.
FIG. 10 is a cross sectional view showing the assembly of a part of the movable cell incubator according to the preferred embodiment of the present invention.
FIG. 11 is a cross sectional view showing a container connected with one of at least one cell culture bag via one of multiple conveying tubes on which a sterile filtering ring is fitted.

### DETAILED DESCRIPTION OF THE PREFERRED EMBODIMENTS

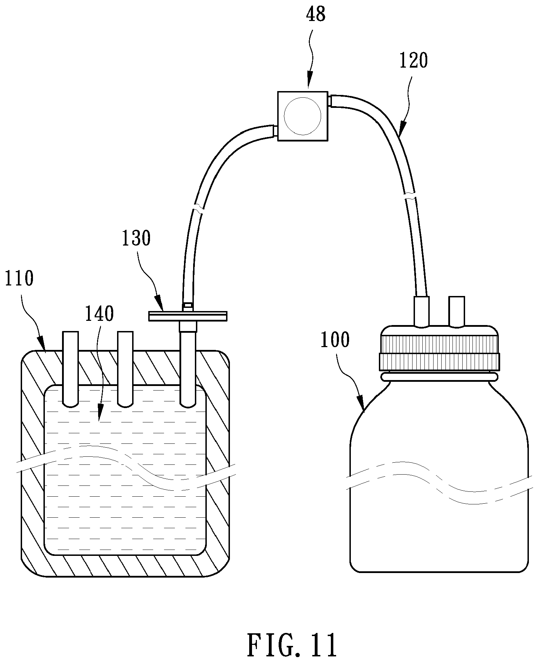

With reference to FIG. 11, a container 100 is connected with one of at least one cell culture bag 110 via one of multiple conveying tubes 120 on which a sterile filtering ring 130 is fitted, thus producing a sterile close system. The sterile close system is movable and is capable of refrigerating the container 100, maintaining temperature, and conveying fluids so as to cultivate cells successfully. The container 100 has a cell culture media 140 filled therein.

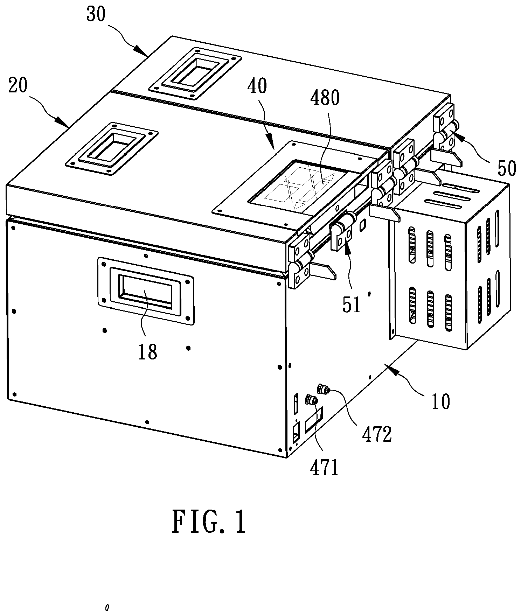

Referring to FIG. 1, a movable cell incubator according to a preferred embodiment of the present invention comprises a body 10, a first lid 20, a second lid 30, and an electric control unit 40.

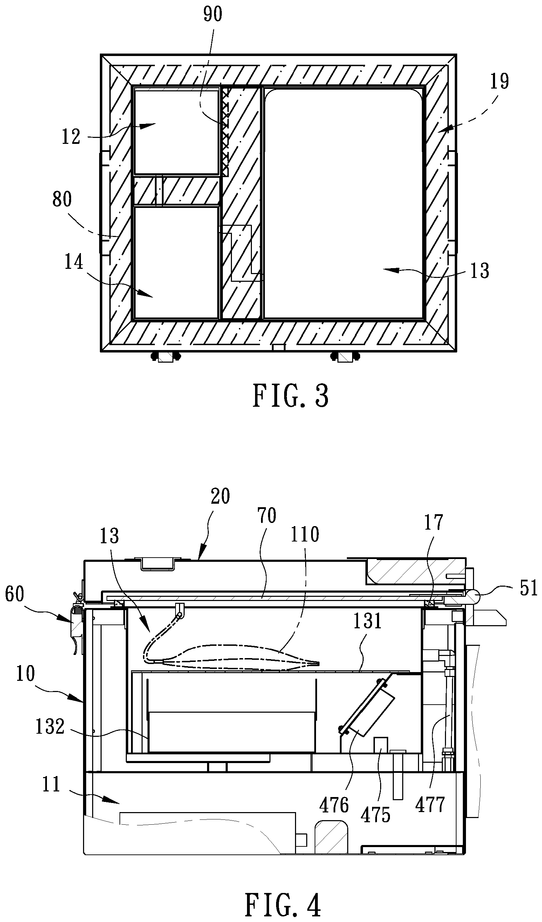

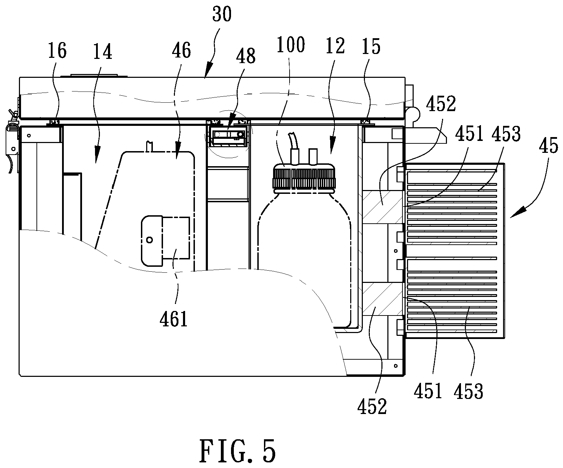

As shown in FIGS. 4 and 5, the body 10 includes a first internal space 11, a refrigeration room 12 and an airtight culture room 13 which are located above the first internal space 11, wherein the refrigeration room 12 is configured to store the container 100, and the culture room 13 accommodates the at least one cell culture bag 110.

The first lid 20 airtightly covers the culture room 13, and the second lid 30 airtightly covers the refrigeration room 12.

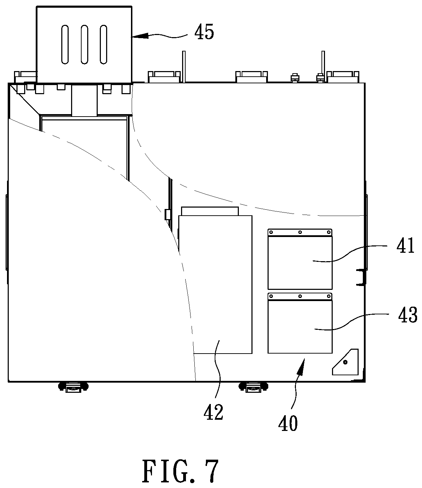

As illustrated in FIGS. 5 to 8, the electric control unit 40 includes a microprocessor 41, a power module 42, a digital/analog conversion module 43 defined between the microprocessor 41 and the power module 42, a heating module 44 for controlling a temperature of the culture room 13, a cooling module 45 configured to supply cold source to the refrigeration room 12, a peristaltic pump module 46 having a step motor 461, a CO₂ detective supply module 47 configured to supply CO₂ to the culture room 13, a flow sensing module 48, and a setting display module 480 exposing and fixed on the first lid 20. The microprocessor 41, the power module 42, and the digital/analog conversion module 43 are accommodated in the first internal space 11 of the body 10. The peristaltic pump module 46 is aseptically connected between the container 100 and the at least one cell culture bag 110 by way of the multiple conveying tubes 120, the flow sensing module 48 is serially connected on the multiple conveying tubes 120, the power module 42 supplies power, and the setting display module 480 is configured to set cell cultivating conditions. The microprocessor 41 operates and controls the cell cultivating conditions, the flow sensing module 48 detects fluid flow, and the peristaltic pump module 46 pumps the cell culture media 140 into the at least one cell culture bag 110 by way of the step motor 461.

With reference to FIG. 5, the cooling module 45 is disposed on an outer wall of the body 10 and includes two chips 451 (Thermoelectric Cooling Chip), two chilling blocks 452 corresponding to the refrigeration room 12, and two heat sink sets 453, wherein the cooling module 45 transfers cold source of the two chips 451 to the refrigeration room 12 via the two chilling blocks 452, and the microprocessor 41 controls a temperature of the refrigeration room 12 at 4 °C to 6 °C.

The body 10 further includes a pump chamber 14 located beside the refrigeration room 12 and the culture room 13 so as to accommodate the peristaltic pump module 46, and the second lid 30 covers the refrigeration room 12 and the pump chamber 14. The refrigeration room 12 has a first seal 15 fixed on a top thereof, and the pump chamber 14 has a second seal 16 mounted on a top thereof so that when the second lid 30 covers the refrigeration room 12 and the pump chamber 14, it matingly contacts with the first seal 15 and the second seal 16. Referring to FIG. 4, between the culture room 13 and the first lid 20 is defined a transparent glass 70, and the culture room 13 has a third seal 17 secured on a top thereof so that when the transparent glass 70 covers the culture room 13, it matingly contacts with the third seal 17 so as to cultivate the cells in an airtight condition.

As shown in FIG. 3, the movable cell incubator further comprises a heat insulating material 80 filled in a second internal space 19 of the refrigeration room 12, the pump chamber 14, and the culture room 13. The heat insulating material 80 is made of polyethylene (PE) so as to insulate heat from the refrigeration room 12, the pump chamber 14, and the culture room 13. Between the refrigeration room 12 and the culture room 13 is defined a vacuum insulation panel 90 configured to attach on an outer wall of the refrigeration room 12, and the vacuum insulation panel 90 has a vacuum protection layer and a core filler filled inside the vacuum protection layer, the vacuum insulation panel 90 is vacuumed and is heat sealed so as to avoid air flowing and heat transferring, thus preventing a low temperature of the refrigeration room 12 influencing the culture room 13.

As shown in FIGS. 1 and 4, the movable cell incubator further comprises plural first connection elements 50, plural second connection elements 51, and multiple locking sets 60 fixed on the first lid 20 and the second lid 30 and locked with the body 10, hence the first lid 20 and the second lid 30 are rotatably connected with the body 10 by way of the plural first connection elements 50 so that the first lid 20 and the second lid 30 open and close the body 10. The transparent glass 70 is rotatably connected with the body 10 by using the plural second connection elements 51 so that the transparent glass 70 and the first lid 20 are individually opened. After the first lid 20 and the second lid 30 cover the body 10, the first lid 20 and the second lid 30 are locked by the multiple locking sets 60.

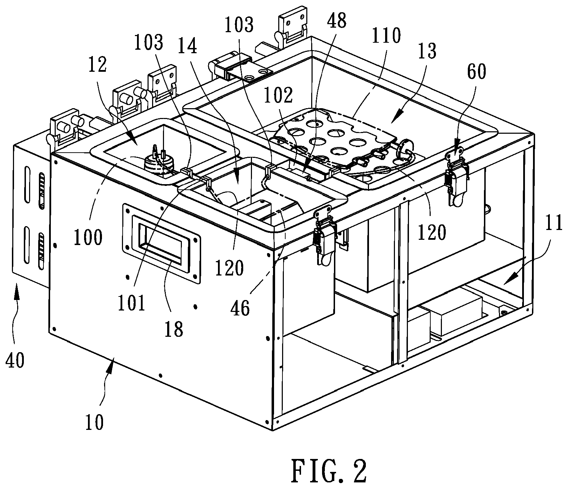

As illustrated in FIG. 2, the body 10 further includes a first through orifice 101, a second through orifice 102, and multiple protective sleeves 103, wherein the first through orifice 101 is in communication with the refrigeration room 12 and the pump chamber 14, the second through orifice 102 communicates with the pump chamber 14 and the culture room 13, and the multiple conveying tubes 120 connect with the peristaltic pump module 46 via the first through orifice 101 from the refrigeration room 12 and extend into the culture room 13 via the second through orifice 102 from the peristaltic pump module 46.

After cultivating the cells, the container 100, the at least one cell culture bag 110, the multiple protective sleeves 103, and the multiple conveying tubes 120 are connected in a sterile environment, and the container 100 is housed in the refrigeration room 12 so that a temperature of the container 100 is maintained at 4 °C to 6 °C, and the multiple conveying tubes 120 are coupled with the peristaltic pump module 46 and are accommodated in the pump chamber 14, then the at least one cell culture bag 110 is placed in the culture room 13, the multiple protective sleeves 103 are fixed on the first through orifice 101 and the second through orifice 102 respectively so that the multiple conveying tubes 120 insert through the first through orifice 101 and the second through orifice 102 of the body 10 by using the multiple protective sleeves 103. Each of the multiple protective sleeves 103 is made of silicone material so as to matingly contact with each of the multiple conveying tubes 120, thus fixing each conveying tube 120 and isolating the refrigeration room 12, the culture room 13, and the pump chamber 14 effectively. Thereafter, the first lid 20 and the second lid 30 airtightly cover the refrigeration room 12, the pump chamber 14 and the culture room 13, and culture time, temperature, and humidity of the cells and output quantity of the peristaltic pump module 46 are set a by way of the setting display module 480.

With reference to FIGS. 2 and 4, the culture room 13 includes a hollow platform 131 defined therein and configured to hold the at least one cell culture bag 110, and the culture room 13 includes a water tray 132 located below the hollow platform 131, wherein the hollow platform 131 communicates with the water tray 132 so that the water tray 132 holds sterile aqueous solution and controls the culture room 13 in a predetermined humidity as cultivating the cells.

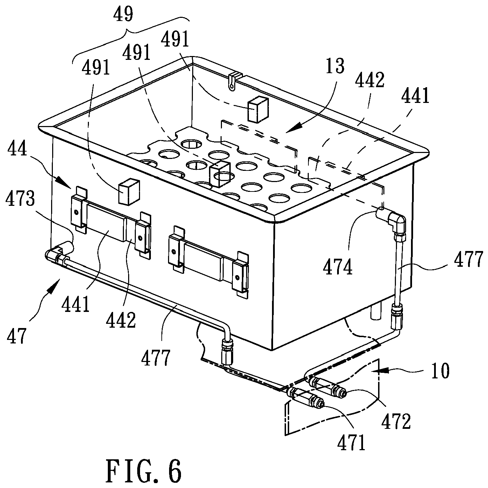

Referring to FIGS. 4 and 6, the CO₂ detective supply module 47 includes an intake valve 471 and an exhaust valve 472 which are arranged on the outer wall of the body 10, a first air hole 473 and a second air hole 474 which are defined on an outer wall of the culture room 13, a CO₂ detector 475 and an air blowing mechanism 476 which are mounted in the culture room 13. The intake valve 471 is in connection with the first air hole 473 via a connection tube 477 so as to guide CO₂ from an external environment, the exhaust valve 472 is coupled with the second air hole 474 via another connection tube 477 so as to release pressure. The air blowing mechanism 476 circulates air in the culture room 13, the CO₂ detector 475 is electrically connected with the microprocessor 41 so as to detect concentration of CO₂ in the culture room 13 and to control the concentration of CO₂ at a predetermined percentage. In this embodiment, the air blowing mechanism 476 is a fan or a blower.

The power module 42 supplies the power to the heating module 44, and the heating module 44 is secured on the outer wall of the culture room 13, wherein the heating module 44 includes multiple resistive heating sheets 441 and plural copper pieces 442 attached on the multiple resistive heating sheets 441 respectively.

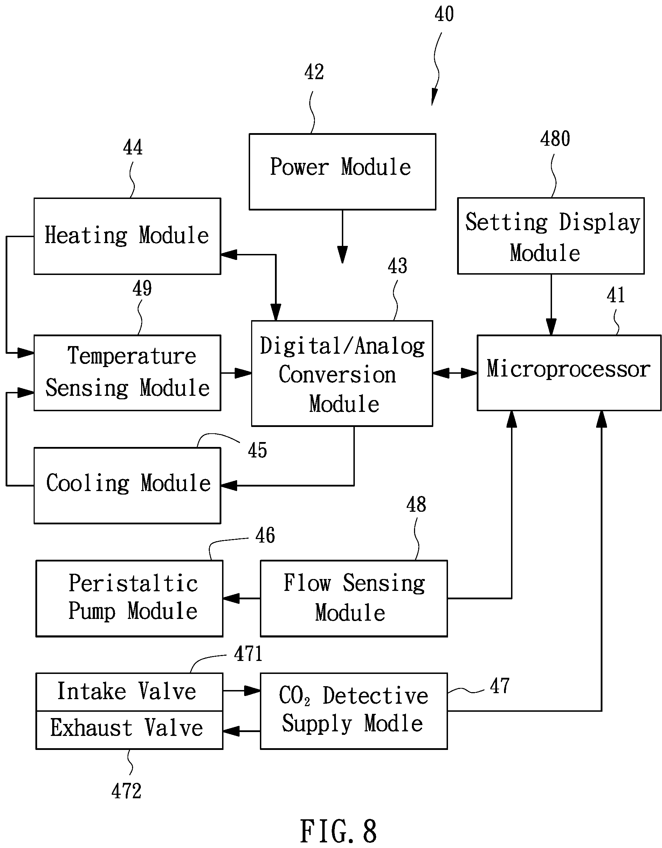

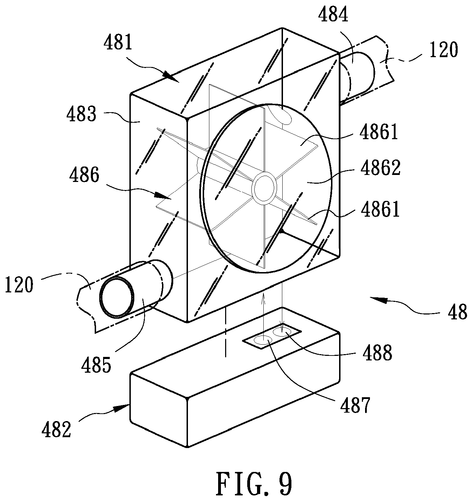

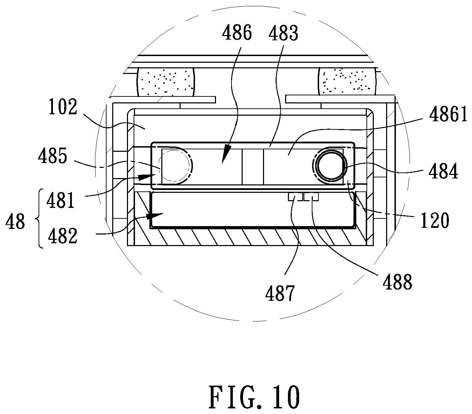

As shown in FIGS. 8 to 10, the flow sensing module 48 is coupled with the microprocessor 41 and includes a flow meter 481 and an infrared transceiver 482, wherein the flow meter 481 has a transparent casing 483, an inlet segment 484 connected to the peristaltic pump module 46, an outlet segment 485 coupled to the at least one cell culture bag 110, and a fan impeller 486 disposed on a central position of the transparent casing 483, wherein the infrared transceiver 482 has a transmitting portion 487 and a receiving portion 488 which face the fan impeller 486. The flow meter 481 is accommodated in the second through orifice 102, the infrared transceiver 482 is retained in a bottom of the second through orifice 102, and the transmitting portion 487 and the receiving portion 488 face the fan impeller 486 of the flow meter 481, hence when the fan impeller 486 rotates, it stops lights projecting to the receiving portion 488. Furthermore, because a distance between any two adjacent of plural blades 4861 of the fan impeller 486 is fixed, between any two adjacent blades 4861 is defined an interspace 4862 configured to accommodate the fluid, and a capacity (cc) of the interspace 4862 is calculated. When the multiple conveying tubes 120 convey the cell culture media 140, the infrared transceiver 482 detects a rotating speed of the fan impeller 486 via a transparent window of the flow meter 481, and after acquiring a rotating speed of the plural blades 4861 and the capacity of the interspace 4862, a message sends back to the microprocessor 41 so that the microprocessor 41 calculates and determines a supply of the cell culture media 140 to the at least one cell culture bag 110, thus controlling delivery quantity of the cell culture media 140 exactly. Preferably, the delivery quantity of the cell culture media 140 is viewed by a liquid crystal display (LCD) of the setting display module 480.

The electric control unit 40 further includes a temperature sensing module 49 electrically connected with the microprocessor 41 and includes multiple sensors 491, wherein each of the multiple sensors 491 is fixed on a central position of the culture room 13 and on the outer wall of the culture room 13 so as to sense exterior temperature and interior temperature of the culture room 13. Thereby, the multiple sensors 491 sense the exterior temperature and the interior temperature of the culture room 13 so as to control the temperature of the culture room 13. When the culture room 13 does not reach to a desired temperature, the microprocessor 41 starts the heating module 44 to heat the culture room 13, thus cultivating the cells at the desired temperature.

Referring to FIGS. 2 and 8, as cultivating the cells in the movable cell incubator, the electric control unit 40 controls the movable cell incubator, and the power module 42 supplies the power. Thereafter, a user sets a desired value by using the setting display module 480 so that the microprocessor 41 has calculating process, the CO₂ detective supply module 47 supplies CO₂, and the concentration of CO₂ in the culture room 13 is set at the predetermined percentage, the heating module 44 heats so that the culture room 13 is controlled at the desired temperature. Thereafter, the cooling module 45 supplies the cold source to the refrigeration room 12 so that the temperature of the refrigeration room 12 maintains at a predetermined value. In addition, the temperature sensing module 49 senses a temperature change and the microprocessor 41 adjusts the cell cultivating conditions randomly. The microprocessor 41 outputs signals and the signals is converted by the digital/analog conversion module 43 so as to change the rotating speed of the step motor 461, and the cell culture media 140 is delivered into the at least one cell culture bag 110 through the multiple conveying tubes 120, thus cultivating the cells in batch metering manner.

As illustrated in FIG. 1, the body 10 further includes a plurality of grips 18 arranged on two opposite ends of the outer wall of the body 10, the first lid 20, and the third lid 30 respectively so as to carry the movable cell incubator and to open and close the first and second lids 20, 30.

Accordingly, the movable cell incubator is compact and is movable easily. Preferably, the movable cell incubator automatically cultivates the cells at designated time and quantity.

The movable cell incubator further has advantages as follows:
1. The setting display module 480 is fixed on the first lid 20 so that the user operates and sets the desired value easily by operating the LCD of the setting display module 480.
2. The first lid 20 and the transparent glass 70 are rotatably connected with the body 10 individually, hence when opening the first lid 20, the user is capable of observing the cells in the body 10 via the transparent glass 70, and the transparent glass 70 presses the third seal 17 so as to keep the airtight condition in the culture room 13.
3. The multiple resistive heating sheets 441 are controlled by the microprocessor 41 and are sensed by the multiple sensors 491 so as to exactly maintain the temperature of the culture room 13.
4. The cell culture media 140 is fed into the at least one cell culture bag 110 at an accurate quantity by way of the step motor 461.
5. The multiple protective sleeves 103 are made of the silicone material and matingly contact with each of the multiple conveying tubes 120, the multiple conveying tubes 120 insert through the pump chamber 14 from the refrigeration room 12 and then extend into the culture room 13 from the pump chamber 14 so as to avoid temperature interferences and to insulate air from the refrigeration room 12, the culture room 13, and the pump chamber 14.
6. The flow sensing module 48 is serially connected on the multiple conveying tubes 120 between the peristaltic pump module 46 and the at least one cell culture bag 110 so as to control the delivery quantity of the cell culture media 140. In addition, the supply of the cell culture media 140 is determined precisely based on the rotating speed of the fan impeller 486 and the capacity of the interspace 4862.

## Claims

1. A movable cell incubator comprising:
a body (10) including a first internal space (11), a refrigeration room (12) and an airtight culture room (13) which are located above the first internal space (11), the refrigeration room (12) being configured to accommodate a cell culture media (140), and the culture room (13) accommodating at least one cell culture bag (110);
a first lid (20) airtightly covering the culture room (13);
a second lid (30) airtightly covering the refrigeration room (12);
an electric control unit (40) including a microprocessor (41), a power module (42), a digital/analog conversion module (43) defined between the microprocessor (41) and the power module (42), a heating module (44) for controlling a temperature of the culture room (13), a cooling module (45) configured to supply cold source to the refrigeration room (12), a peristaltic pump module (46), a CO₂ detective supply module (47) configured to supply CO₂ to the culture room (13) and a setting display module (480) exposed and fixed on the first lid (20);
wherein the peristaltic pump module (46) is aseptically connected between the cell culture media (140) and the at least one cell culture bag (110) by way of multiple conveying tubes (120), the power module (42) supplies power, and the setting display module (480) is configured to set cell cultivating conditions, the microprocessor (41) operates and controls the cell cultivating conditions and the peristaltic pump module (46) pumps the cell culture media (140) into the at least one cell culture bag (110);
**characterized in that**
the electric control unit (40) further includes a flow sensing module (48), the flow sensing module (48) being serially connected on the multiple conveying tubes (120), wherein the flow sensing module (48) is configured to detect fluid flow, wherein the flow sensing module (48) is coupled with the microprocessor (41) and includes a flow meter (481) and an infrared transceiver (482), wherein the flow meter (481) has a transparent casing (483), an inlet segment (484) connected to the peristaltic pump module (46), an outlet segment (485) coupled to the at least one cell culture bag (110), and a fan impeller (486) disposed on a central position of the transparent casing (483), wherein the infrared transceiver (482) is configured to detect a rotating speed of the fan impeller (486) and has a transmitting portion (487) and a receiving portion (488) which face the fan impeller (486).

2. The movable cell incubator as claimed in claim 1, wherein the body (10) further includes a pump chamber (14) located beside the refrigeration room (12) and the culture room (13) so as to accommodate the peristaltic pump module (46), and the second lid (30) covers the refrigeration room (12) and the pump chamber (14), the refrigeration room (12) has a first seal (15) fixed on a top thereof, and the pump chamber (14) has a second seal (16) mounted on a top thereof so that when the second lid (30) covers the refrigeration room (12) and the pump chamber (14), the second lid (30) matingly contacts with the first seal (15) and the second seal (16).

3. The movable cell incubator as claimed in claim 2, wherein the body (10) further includes a first through orifice (101) and a second through orifice (102), the first through orifice (101) is in communication with the refrigeration room (12) and the pump chamber (14), the second through orifice (102) communicates with the pump chamber (14) and the culture room (13), and the multiple conveying tubes (120) connect with the peristaltic pump module (46) via the first through orifice (101) from the refrigeration room (12) and extend into the culture room (13) via the second through orifice (102) from the peristaltic pump module (46).

4. The movable cell incubator as claimed in claim 3, further comprising multiple protective sleeves (103) respectively fitted on the multiple conveying tubes (120) and fixed on the first through orifice (101) and the second through orifice (102) individually so that the multiple conveying tubes (120) insert through the first through orifice (101) and the second through orifice (102) of the body (10) by using the multiple protective sleeves (103), wherein each of the multiple protective sleeves (103) is made of silicone material.

5. The movable cell incubator as claimed in claim 1, wherein the culture room (13) includes a hollow platform (131) defined therein and configured to hold the at least one cell culture bag (110), and the culture room (13) includes a water tray (132) located below the hollow platform (131).

6. The movable cell incubator as claimed in claim 1, wherein between the culture room (13) and the first lid (20) is defined a transparent glass (70), and the culture room (13) has a third seal (17) secured on a top thereof so that when the transparent glass (70) covers the culture room (13), it matingly contacts with the third seal (17).

7. The movable cell incubator as claimed in claim 6, wherein the first lid (20) is rotatably connected with the body (10) by way of plural first connection elements (50), and the transparent glass (70) is rotatably connected with the body (10) by using plural second connection elements (51) so that the transparent glass (70) and the first lid (20) are individually opened.

8. The movable cell incubator as claimed in claim 1, wherein the cooling module (45) is disposed on an outer wall of the body (10) and includes at least one chip (451), at least one chilling block (452) corresponding to the refrigeration room (12), and at least one heat sink set (453), wherein the cooling module (45) transfers cold source of the at least one chip (451) to the refrigeration room (12) via the at least one chilling block (452).

9. The movable cell incubator as claimed in claim 1, wherein the power module (42) supplies the power to the heating module (44), and the heating module (44) is secured on an outer wall of the culture room (13), wherein the heating module (44) includes multiple resistive heating sheets (441) and plural copper pieces (442) attached on the multiple resistive heating sheets (441) respectively.

10. The movable cell incubator as claimed in claim 1, wherein the CO₂ detective supply module (47) includes an intake valve (471) and an exhaust valve (472) which are arranged on the outer wall of the body (10), a first air hole (473) and a second air hole (474) which are defined on the outer wall of the culture room (13), a CO₂ detector (475) and an air blowing mechanism (476) which are mounted in the culture room (13), the intake valve(471) is in connection with the first air hole (473) via a connection tube (477) so as to guide CO₂ from an external environment, the exhaust valve (472) is coupled with the second air hole (474) via another connection tube (477) so as to release pressure, the air blowing mechanism (476) circulates air in the culture room (13), and the CO₂ detector (475) is electrically connected with the microprocessor (41) so as to detect concentration of CO₂ in the culture room (13).

11. The movable cell incubator as claimed in claim 1, further comprising a temperature sensing module (49) electrically connected with the microprocessor (41), and the temperature sensing module (49) including multiple sensors (491), wherein each of the multiple sensors (491) is fixed on a central position of the culture room (13) and on the outer wall of the culture room (13) so as to sense exterior temperature and interior temperature of the culture room (13).

12. The movable cell incubator as claimed in claim 1, further comprising plural first connection elements (50) and multiple locking sets (60) fixed on the first lid (20) and the second lid (30) and locked with the body (10), hence the first lid (20) and the second lid (30) are rotatably connected with the body (10) by way of the plural first connection elements (50), and when the first lid (20) and the second lid (30) cover the body (10), the first lid (20) and the second lid (30) are locked by the multiple locking sets (60).

13. The movable cell incubator as claimed in claim 1, further comprising a heat insulating material (80) filled in a second internal space (19) of the refrigeration room (12), the pump chamber (14), and the culture room (13).

14. The movable cell incubator as claimed in claim 1, further comprising a vacuum insulation panel (90) configured to attach on an outer wall of the refrigeration room (12).

## Patentansprüche

1. Beweglicher Zellinkubator umfassend:
einen Körper (10), welcher einen ersten Innenraum (11), einen Kühlraum (12) und einen luftdichten Kulturraum (13), welche oberhalb des ersten Innenraums (11) angeordnet sind, enthält, wobei der Kühlraum (12) zur Aufnahme eines Zellkulturmediums (140) ausgebildet ist, wobei der Kulturraum (13) mindestens einen Zellkulturbeutel (110) aufnimmt,
einen ersten Deckel (20), welcher den Kulturraum (13) luftdicht abdeckt, einen zweiten Deckel (30), welcher den Kühlraum (12) luftdicht abdeckt, eine elektrische Steuereinheit (40) umfassend einen Mikroprozessor (41), ein Leistungsmodul (42), ein Digital/Analog-Wandlermodul (43), welches zwischen dem Mikroprozessor (41) und dem Leistungsmodul (42) definiert ist, ein Heizmodul (44) zur Kontrolle einer Temperatur des Kulturraums (13), ein Kühlmodul (45), welches so konfiguriert ist, dass es dem Kühlraum (12) eine Kältequelle zuführt, ein peristaltisches Pumpenmodul (46), ein CO₂- Detektor - Versorgungsmodul (47), welches so konfiguriert ist, dass es CO₂ in den Kulturraum (13) liefert, sowie ein Einstellanzeigemodul (480), welches am ersten Deckel (20) exponiert und befestigt ist,
wobei das peristaltische Pumpenmodul (46) mit den Zellkulturmedien (140) und dem mindestens einen Zellkulturbeutel (110) über mehrere Förderschläuche (120) aseptisch verbunden ist, wobei das Leistungsmodul (42) Strom liefert, wobei das Einstellanzeigemodul (480) zur Einstellung von Zellkultivierungsbedingungen konfiguriert ist, wobei der Mikroprozessor (41) die Zellkultivierungsbedingungen steuert und regelt und das peristaltische Pumpenmodul (46) das Zellkulturmedium (140) in den mindestens einen Zellkulturbeutel (110) pumpt,
**dadurch gekennzeichnet,**
**dass** die elektrische Steuereinheit (40) ferner ein Durchflusserfassungsmodul (48) umfasst, wobei das Durchflusserfassungsmodul (48) an den mehreren Förderrohren (120) in Reihe geschaltet ist, wobei das Durchflusserfassungsmodul (48) so konfiguriert ist, dass es einen Fluidstrom erfasst, wobei das Durchflusserfassungsmodul (48) mit dem Mikroprozessor (41) gekoppelt ist und einen Durchflussmesser (481) und einen Infrarotsendeempfänger (482) umfasst, wobei der Durchflussmesser (481) ein transparentes Gehäuse (483), ein Einlasssegment (484), welches mit dem peristaltischen Pumpenmodul (46) verbunden ist, ein Auslasssegment (485), welches mit dem mindestens einen Zellkulturbeutel (110) gekoppelt ist, sowie ein Gebläserad (486) aufweist, welches an einer zentralen Position des transparenten Gehäuses (483) angeordnet ist, wobei der Infrarotsendeempfänger (482) so konfiguriert ist, dass er eine Drehgeschwindigkeit des Gebläserads (486) erfasst, sowie einen Sendeabschnitt (487) und einen Empfangsabschnitt (488) aufweist, welche dem Gebläserad (486) zugewandt sind.

2. Beweglicher Zellinkubator nach Anspruch 1, bei welchem der Körper (10) ferner eine Pumpenkammer (14) umfasst, welche neben dem Kühlraum (12) und dem Kulturraum (13) angeordnet ist, um das peristaltische Pumpenmodul (46) aufzunehmen, wobei der zweite Deckel (30) den Kühlraum (12) und die Pumpenkammer (14) abdeckt, wobei der Kühlraum (12) eine erste Dichtung (15) aufweist, welche an einer Oberseite desselben befestigt ist, wobei die Pumpenkammer (14) eine zweite Dichtung (16) aufweist, welche an einer Oberseite desselben befestigt ist, so dass, wenn der zweite Deckel (30) den Kühlraum (12) und die Pumpenkammer (14) abdeckt, der zweite Deckel (30) mit der ersten Dichtung (15) und der zweiten Dichtung (16) passend in Kontakt steht.

3. Beweglicher Zellinkubator nach Anspruch 2, bei welchem der Körper (10) ferner eine erste Durchgangsöffnung (101) und eine zweite Durchgangsöffnung (102) aufweist, wobei die erste Durchgangsöffnung (101) mit dem Kühlraum (12) und der Pumpenkammer (14) in Verbindung steht, wobei die zweite Durchgangsöffnung (102) mit der Pumpenkammer (14) und dem Kulturraum (13) in Verbindung steht, wobei die mehrfachen Förderschläuche (120) mit dem peristaltischen Pumpenmodul (46) über die erste Durchgangsöffnung (101) vom Kühlraum (12) aus verbunden sind und sich über die zweite Durchgangsöffnung (102) vom peristaltischen Pumpenmodul (46) aus in den Kulturraum (13) erstrecken.

4. Beweglicher Zellinkubator nach Anspruch 3, welcher ferner mehrere Schutzhülsen (103) umfasst, die jeweils auf die mehreren Förderschläuche (120) aufgesetzt und einzeln an der ersten Durchgangsöffnung (101) und der zweiten Durchgangsöffnung (102) befestigt sind, so dass die mehreren Förderschläuche (120) unter Verwendung der mehreren Schutzhülsen (103) durch die erste Durchgangsöffnung (101) und die zweite Durchgangsöffnung (102) des Körpers (10) eingeführt werden, wobei jede der mehreren Schutzhülsen (103) aus Silikonmaterial hergestellt ist.

5. Beweglicher Zellinkubator nach Anspruch 1, bei welchem der Kulturraum (13) eine hohle Plattform (131) umfasst, welche darin definiert und konfiguriert ist, um den mindestens einen Zellkulturbeutel (110) zu halten, wobei der Kulturraum (13) eine Wasserwanne (132) umfasst, welche unterhalb der hohlen Plattform (131) angeordnet ist.

6. Beweglicher Zellinkubator nach Anspruch 1, bei welchem zwischen dem Kulturraum (13) und dem ersten Deckel (20) ein transparentes Glas (70) definiert ist, wobei der Kulturraum (13) eine dritte Dichtung (17) aufweist, welche an einer Oberseite desselben befestigt ist, so dass, wenn das transparente Glas (70) den Kulturraum (13) abdeckt, es passend mit der dritten Dichtung (17) in Kontakt steht.

7. Beweglicher Zellinkubator nach Anspruch 6, bei welchem der erste Deckel (20) drehbar mit dem Körper (10) über mehrere erste Verbindungselemente (50) verbunden ist, wobei das transparente Glas (70) drehbar mit dem Körper (10) durch mehrere zweite Verbindungselemente (51) verbunden ist, so dass das transparente Glas (70) und der erste Deckel (20) einzeln geöffnet werden können.

8. Beweglicher Zellinkubator nach Anspruch 1, bei welchem das Kühlmodul (45) an einer Außenwand des Körpers (10) angeordnet ist und mindestens einen Chip (451), mindestens einen dem Kühlraum (12) entsprechenden Kühlblock (452) und mindestens einen Wärmesenkensatz (453) aufweist, wobei das Kühlmodul (45) die Kältequelle des mindestens einen Chips (451) über den mindestens einen Kühlblock (452) an den Kühlraum (12) überträgt.

9. Beweglicher Zellinkubator nach Anspruch 1, bei welchem das Leistungsmodul (42) das Heizmodul (44) mit Strom versorgt und das Heizmodul (44) an einer Außenwand des Kulturraums (13) befestigt ist, wobei das Heizmodul (44) mehrere Widerstandsheizplatten (441) und mehrere Kupferstücke (442) aufweist, welche entsprechend an den mehreren Widerstandsheizplatten (441) befestigt sind.

10. Beweglicher Zellinkubator nach Anspruch 1, bei welchem das CO₂ - Detektorversorgungsmodul (47) ein Einlassventil (471) und ein Auslassventil (472), welche an der Außenwand des Körpers (10) angeordnet sind, ein erstes Luftloch (473) und ein zweites Luftloch (474), welche an der Außenwand des Kulturraums (13) definiert sind, sowie einen CO₂-Detektor (475) und einen Luftblasmechanismus (476), welche in dem Kulturraum (13) angebracht sind, umfasst, wobei das Einlassventil (471) mit dem ersten Luftloch (473) über ein Verbindungsrohr (477) in Verbindung steht, um CO₂ aus einer äußeren Umgebung zu leiten, wobei das Auslassventil (472) mit dem zweiten Luftloch (474) über ein weiteres Verbindungsrohr (477) gekoppelt ist, um Druck abzulassen, wobei der Luftblasmechanismus (476) die Luft in dem Kulturraum (13) zirkulieren lässt, wobei der CO₂ - Detektor (475) elektrisch mit dem Mikroprozessor (41) verbunden ist, um die Konzentration von CO₂ in dem Kulturraum (13) zu erfassen.

11. Beweglicher Zellinkubator nach Anspruch 1, ferner umfassend ein Temperaturerfassungsmodul (49), welches elektrisch mit dem Mikroprozessor (41) verbunden ist, wobei das Temperaturerfassungsmodul (49) mehrere Sensoren (491) enthält, wobei jeder der mehreren Sensoren (491) an einer zentralen Position des Kulturraums (13) und an der Außenwand des Kulturraums (13) befestigt ist, um die Außentemperatur und die Innentemperatur des Kulturraums (13) zu erfassen.

12. Beweglicher Zellinkubator nach Anspruch 1, ferner mit mehreren ersten Verbindungselementen (50) und mehreren Verriegelungssätzen (60), welche an dem ersten Deckel (20) und dem zweiten Deckel (30) befestigt und mit dem Körper (10) verriegelt sind, so dass der erste Deckel (20) und der zweite Deckel (30) über die mehreren ersten Verbindungselemente (50) drehbar mit dem Körper (10) verbunden sind, wobei wenn der erste Deckel (20) und der zweite Deckel (30) den Körper (10) abdecken, der erste Deckel (20) und der zweite Deckel (30) durch die mehreren Verriegelungssätze (60) verriegelt sind.

13. Beweglicher Zellinkubator nach Anspruch 1, ferner umfassend ein wärmeisolierendes Material (80), welches in einen zweiten Innenraum (19) des Kühlraums (12), der Pumpenkammer (14) und des Kulturraums (13) gefüllt ist.

14. Beweglicher Zellinkubator nach Anspruch 1, ferner umfassend ein Vakuumisolationspanel (90), welches zur Anbringung an einer Außenwand des Kühlraums (12) konfiguriert ist.

## Revendications

1. Incubateur de cellules mobiles comprenant:
un corps (10) incluant un premier espace interne (11), une salle de réfrigération (12) et une salle de culture (13) étanche à l'air qui sont situées au-dessus du premier espace interne (11), la salle de réfrigération (12) étant configurée pour loger un milieu de culture cellulaire (140), et la salle de culture (13) logeant au moins un sac de culture cellulaire (110);
un premier couvercle (20) recouvrant de manière étanche à l'air la salle de culture (13);
un second couvercle (30) recouvrant de manière étanche à l'air la salle de réfrigération (12);
une unité de commande électrique (40) incluant un microprocesseur (41), un module d'énergie (42), un module de conversion numérique/analogique (43) défini entre le microprocesseur (41) et le module d'énergie (42), un module de chauffage (44) pour commander une température de la salle de culture (13), un module de refroidissement (45) configuré pour fournir une source froide à la salle de réfrigération (12), un module de pompe péristaltique (46), un module de fourniture de détection de CO₂ (47) configuré pour fournir du CO₂ à la salle de culture (13) et un module d'affichage de réglage (480) exposé et fixé sur le premier couvercle (20);
dans lequel le module de pompe péristaltique (46) est connecté de manière aseptique entre le milieu de culture cellulaire (140) et l'au moins un sac de culture cellulaire (110) au moyen de multiples tubes de transport (120), le module d'énergie (42) fournit de l'énergie, et le module d'affichage de réglage (480) est configuré pour régler des conditions de culture cellulaire, le microprocesseur (41) pilote et commande les conditions de culture cellulaire et le module de pompe péristaltique (46) pompe le milieu de culture cellulaire (140) dans l'au moins un sac de culture cellulaire (110);
**caractérisé en ce que**
l'unité de commande électrique (40) inclut en outre un module de détection d'écoulement (48), le module de détection d'écoulement (48) étant connecté en série sur les multiples tubes de transport (120), dans lequel le module de détection d'écoulement (48) est configuré pour détecter un écoulement de fluide, dans lequel le module de détection d'écoulement (48) est couplé au microprocesseur (41) et inclut un débitmètre (481) et un émetteur-récepteur infrarouge (482), dans lequel le débitmètre (481) présente un boîtier transparent (483), un segment d'entrée (484) connecté au module de pompe péristaltique (46), un segment de sortie (485) couplé à l'au moins un sac de culture cellulaire (110), et une roue de ventilateur (486) disposée sur une position centrale du boîtier transparent (483), dans lequel l'émetteur-récepteur infrarouge (482) est configuré pour détecter une vitesse de rotation de la roue de ventilateur (486) et présente une partie de transmission (487) et une partie de réception (488) qui sont face à la roue de ventilateur (486).

2. Incubateur de cellules mobiles selon la revendication 1, dans lequel le corps (10) inclut en outre une chambre de pompage (14) située à côté de la salle de réfrigération (12) et la salle de culture (13) de façon à loger le module de pompe péristaltique (46), et le second couvercle (30) recouvre la salle de réfrigération (12) et la chambre de pompage (14), la salle de réfrigération (12) présente un premier joint d'étanchéité (15) fixé sur une partie supérieure de celle-ci, et la chambre de pompage (14) présente un deuxième joint d'étanchéité (16) monté sur une partie supérieure de celle-ci de sorte que lorsque le second couvercle (30) recouvre la salle de réfrigération (12) et la chambre de pompage (14), le second couvercle (30) vient en contact par accouplement avec le premier joint d'étanchéité (15) et le deuxième joint d'étanchéité (16).

3. Incubateur de cellules mobiles selon la revendication 2, dans lequel le corps (10) inclut en outre un premier orifice traversant (101) et un second orifice traversant (102), le premier orifice traversant (101) est en communication avec la salle de réfrigération (12) et la chambre de pompage (14), le second orifice traversant (102) communique avec la chambre de pompage (14) et la salle de culture (13), et les multiples tubes de transport (120) sont connectés au module de pompe péristaltique (46) par l'intermédiaire du premier orifice traversant (101) à partir de la salle de réfrigération (12) et s'étendent dans la salle de culture (13) par l'intermédiaire du second orifice traversant (102) à partir du module de pompe péristaltique (46).

4. Incubateur de cellules mobiles selon la revendication 3, comprenant en outre de multiples manchons de protection (103) respectivement ajustés sur les multiples tubes de transport (120) et fixés sur le premier orifice traversant (101) et le second orifice traversant (102) individuellement de sorte que les multiples tubes de transport (120) s'insèrent à travers le premier orifice traversant (101) et le second orifice traversant (102) du corps (10) en utilisant les multiples manchons de protection (103), dans lequel chacun des multiples manchons de protection (103) est fait de matériau en silicone.

5. Incubateur de cellules mobiles selon la revendication 1, dans lequel la salle de culture (13) inclut une plateforme creuse (131) définie dans celle-ci et configurée pour maintenir l'au moins un sac de culture cellulaire (110), et la salle de culture (13) inclut un bac à eau (132) situé en dessous de la plateforme creuse (131).

6. Incubateur de cellules mobiles selon la revendication 1, dans lequel entre la salle de culture (13) et le premier couvercle (20) est défini un verre transparent (70), et la salle de culture (13) présente un troisième joint d'étanchéité (17) fixé solidement sur une partie supérieure de celle-ci de sorte que lorsque le verre transparent (70) recouvre la salle de culture (13), il vient en contact par accouplement avec le troisième joint d'étanchéité (17).

7. Incubateur de cellules mobiles selon la revendication 6, dans lequel le premier couvercle (20) est connecté de manière rotative au corps (10) au moyen de plusieurs premiers éléments de connexion (50), et le verre transparent (70) est connecté de manière rotative au corps (10) en utilisant plusieurs seconds éléments de connexion (51) de sorte que le verre transparent (70) et le premier couvercle (20) sont individuellement ouverts.

8. Incubateur de cellules mobiles selon la revendication 1, dans lequel le module de refroidissement (45) est disposé sur une paroi externe du corps (10) et inclut au moins une puce (451), au moins un bloc de refroidissement (452) correspondant à la salle de réfrigération (12), et au moins un ensemble de dissipateur thermique (453), dans lequel le module de refroidissement (45) transfère la source froide de l'au moins une puce (451) à la salle de réfrigération (12) par l'intermédiaire de l'au moins un bloc de refroidissement (452).

9. Incubateur de cellules mobiles selon la revendication 1, dans lequel le module d'énergie (42) fournit l'énergie au module de chauffage (44), et le module de chauffage (44) est fixé solidement sur une paroi externe de la salle de culture (13), dans lequel le module de chauffage (44) inclut de multiples feuilles de chauffage résistives (441) et plusieurs pièces de cuivre (442) appliquées sur les multiples feuilles de chauffage résistives (441) respectivement.

10. Incubateur de cellules mobiles selon la revendication 1, dans lequel le module de fourniture de détection de CO₂ (47) inclut une soupape d'admission (471) et une soupape d'échappement (472) qui sont agencées sur la paroi externe du corps (10), un premier trou d'air (473) et un second trou d'air (474) qui sont définis sur la paroi externe de la salle de culture (13), un détecteur de CO₂ (475) et un mécanisme de soufflage d'air (476) qui sont montés dans la salle de culture (13), la soupape d'admission (471) est en connexion avec le premier trou d'air (473) par l'intermédiaire d'un tube de connexion (477) de façon à guider le CO₂ à partir d'un environnement externe, la soupape d'échappement (472) est couplée au second trou d'air (474) par l'intermédiaire d'un autre tube de connexion (477) de façon à libérer la pression, le mécanisme de soufflage d'air (476) fait circuler de l'air dans la salle de culture (13), et le détecteur de CO₂ (475) est connecté électriquement au microprocesseur (41) de façon à détecter la concentration de CO₂ dans la salle de culture (13).

11. Incubateur de cellules mobiles selon la revendication 1, comprenant en outre un module de détection de température (49) connecté électriquement au microprocesseur (41), et le module de détection de température (49) incluant de multiples capteurs (491), dans lequel chacun des multiples capteurs (491) est fixé sur une position centrale de la salle de culture (13) et sur la paroi externe de la salle de culture (13) de façon à détecter la température extérieure et la température intérieure de la salle de culture (13).

12. Incubateur de cellules mobiles selon la revendication 1, comprenant en outre plusieurs premiers éléments de connexion (50) et de multiples ensembles de verrouillage (60) fixés sur le premier couvercle (20) et le second couvercle (30) et verrouillés avec le corps (10), par conséquent le premier couvercle (20) et le second couvercle (30) sont connectés de manière rotative au corps (10) au moyen des plusieurs premiers éléments de connexion (50), et lorsque le premier couvercle (20) et le second couvercle (30) recouvrent le corps (10), le premier couvercle (20) et le second couvercle (30) sont verrouillés par les multiples ensembles de verrouillage (60).

13. Incubateur de cellules mobiles selon la revendication 1, comprenant en outre un matériau d'isolation thermique (80) chargé dans un second espace interne (19) de la salle de réfrigération (12), la chambre de pompage (14), et la salle de culture (13).

14. Incubateur de cellules mobiles selon la revendication 1, comprenant en outre un panneau d'isolation sous vide (90) configuré pour s'appliquer sur une paroi externe de la salle de réfrigération (12).
